# EUROPEAN PATENT SPECIFICATION

(11) **EP 2 961 666 B1**
(45) Date of publication and mention of the grant of the patent: **09.01.2019**
(21) Application number: 14757321.6
(22) Date of filing: 28.02.2014
(51) Int. Cl.: B65D 85/00, A61J 1/00

(54) **CONTAINERS WITH INSERTS**
BEHÄLTER MIT EINSÄTZEN
CONTENANT POURVU D'ÉLÉMENTS INSÉRABLES

(30) Priority: 01.03.2013 US 201361771194 P
(43) Date of publication of application: 06.01.2016
(73) Proprietor: CSP Technologies, Inc., Auburn, Alabama 36832 (US)
(72) Inventor: LUCAS, Franklin, Lee, Jr., Opeliak, AL 36804 (US); HUNT, Benjamin, Auburn, AL 36830 (US); ROGERS, Joseph, W., Lafayette Hill, PA 19444 (US); FREEDMAN, Jonathan, Auburn, AL 36830 (US); BELFANCE, John, Phenix City, AL 36867 (US)
(74) Representative: Potter Clarkson LLP
(86) International application number: PCT/US2014/019429
(87) International publication number: WO 2014/134455

(56) References cited:
- DE-A1-102007 016 937
- US-A- 3 878 968
- US-A- 4 387 802
- US-A- 4 464 552
- US-A- 4 961 496
- US-A1- 2011 127 269
- US-A1- 2012 080 330
- US-B1- 6 726 006

## Description

### BACKGROUND OF THE INVENTION

### Field of Invention

The invention relates generally to containers that can be used, for example, to house a plurality of flat, elongate articles such as blood glucose test strips or other diagnostic strips. More particularly, the invention relates to inserts within containers that provide improved organization and accessibility of, e.g., blood glucose test strips, especially for persons with swollen or otherwise debilitated fingers, e.g., diabetics.

### Description of Related Art

Diabetes is a chronic condition in which a person has elevated blood glucose levels that result from defects in the body's ability to produce and/or use insulin. It is estimated that tens of millions of people in the United States alone suffer from diabetes and hundreds of millions of people around the world suffer from the disease. Unfortunately, the incidence of diabetes is increasing. Without treatment, diabetes can lead to severe complications such as heart disease, stroke, blindness, kidney failure, amputations, and death related to pneumonia and flu.

Management of diabetes is complex as the level of blood glucose entering the bloodstream is dynamic. To properly manage diabetes, patients must, among other things, carefully monitor their blood glucose levels. Daily diagnostic information, such as blood glucose, is typically obtained from a capillary blood sample with a lancing device. The blood sample is typically disposed onto a portion of a blood glucose test strip, which is then inserted into a port of a blood glucose meter for measurement. This type of monitoring is typically necessary before meals and helps to regulate proper insulin intake.

In addition to the potential diabetes-related complications listed above, diabetes can also affect one's skin and increase the risk of problems with one's extremities. Diabetes patients often experience debilitating musculoskeletal changes, including joint pain, stiffness, swelling, and nodules under the skin, particularly in the fingers.

Blood glucose test strips are typically stored and sold in small, tightly sealed, environmentally controlled containers in quantities of about 25 or 50 strips per container. Tight seals, e.g., moisture tight seals, are important to preserve the useful life of blood glucose test strips, which may be adversely affected by moisture and other elements present in the ambient environment. These containers must be large enough to ensure that the blood glucose test strips are not damaged and that the strips do not interfere with the sealing integrity of the storage container. Current blood glucose test strip containers are often problematic for diabetics who have swelling, stiffness and/or pain in their fingers. The strips may be difficult to remove, especially one at a time, with a diabetic's swollen fingers. A user may need to compensate for the difficulty in retrieving a strip by tilting the container or shaking and pouring out the contents, which may cause the blood glucose test strips to become damaged, contaminated or lost. The ends of the vertically-disposed strips are typically located well below the opening of the container, and can thus be difficult to access for a diabetic, especially one strip at a time.

Thus, there is a need for environmentally controlled (e.g., moisture tight) containers that provide easier access to flat elongate articles, such as blood glucose test strips, especially for people with swollen fingers, e.g., diabetics.

US-A-2012/080330 discloses analyte test strip containers, and methods for their manufacture. The analyte test strip containers may include an interior side of a base that varies in height. Inserts provide the cavity floor with a varying height with respect to a longitudinal axis of the container.

US-A-2011/0127269 discloses a test strip container with a lid having a vaulted underside in which the strips project when the container is closed.

DE-A-10 2007 016 937 discloses a container for storing packaged goods, in particular test strips, comprising a housing with an opening and a lid for closing the opening, wherein the housing receives an inner body which can move relative to the housing and which can move in the direction of the opening. In order for the packaged goods to be easily gripped regardless of the fill level of the container, the container is characterized by attachment means which connect the lid and the inner body to each other in such a manner that the inner body is pulled in the direction of the opening when the lid is opened.

### BRIEF SUMMARY OF THE INVENTION

According to the invention, there is provided a container assembly as defined in the appended Claim 1.

Also disclosed herein are inserts for container assemblies, which facilitate the organization and configuration of arrays of flat elongate articles. Inserts may hold such articles in a generally vertical (optionally, slightly slanted) storage position and facilitate accessibility of the articles for extraction, e.g., one at a time, especially for persons with debilitated fingers, e.g., diabetics.

### BRIEF DESCRIPTION OF THE DRAWINGS

The invention will be described in conjunction with the following drawings in which like reference numerals designate like elements and wherein:
FIG. 1 is a perspective view of a first embodiment of a container and insert assembly according to the invention, in an opened position;
FIG. 2 is a cross sectional view of the assembly of FIG. 1, in a closed position;
FIG. 3 is not an embodiment of the invention and shows a perspective view of a container and insert assembly, in an opened position;
FIG. 4 is not an embodiment of the invention and shows a cross sectional view of the assembly of FIG. 3, in a closed position;
FIG. 5 is not an embodiment of the invention and shows a perspective view of container and insert assembly in an opened position;
FIG. 6 is a cross sectional view of the assembly of FIG. 5, in a closed position;
FIG. 7 is not an embodiment of the invention and shows a perspective view of a container and insert assembly in an opened position;
FIG. 8 is a cross sectional view of the assembly of FIG. 7, in a closed position;
FIG. 9 is not an embodiment of the invention and shows a container and insert assembly in an opened position;
FIG. 10 is a cross sectional view of the assembly of FIG. 9, in a closed position;
FIG. 11 is not an embodiment of the invention and shows a container and insert assembly in an opened position;
FIG. 12 is a perspective view of the assembly of FIG. 11 in the opened position, with the container partially hidden to show features of the insert;
FIG. 13 is not an embodiment of the invention and shows a cross sectional view of a container and insert assembly in an opened position;
FIG. 14 is a perspective view of the assembly of FIG. 13, in the opened position;
FIG. 15 is a perspective view of the assembly of FIG. 13 in the opened position, with the container partially hidden from view to show features of the insert;
FIG. 16 is not an embodiment of the invention and shows a perspective view of a container and insert assembly in an opened position;
FIG. 17 is a cross sectional view of the assembly of FIG. 16, in a closed position;
FIG. 18 is a cross sectional view of the assembly of FIG. 1;
FIG. 19 is a cross sectional view of the assembly of FIG. 5;
FIG. 20 is a cross sectional view of the assembly of FIG. 7;
FIG. 21 is a cross sectional view of the assembly of FIG. 9;
FIG. 22 is a perspective view of the assembly of FIG. 13 in the opened position, with the container partially hidden from view to show features of the insert;
FIG. 23 is a perspective view of the assembly of FIG. 16;
FIG. 24 is a cross sectional view of the assembly of FIG. 16;
FIGS. 25-29 are perspective views of various components of the assembly of FIG. 16;
FIG. 30 is not an embodiment of the invention and shows a perspective view of a container and insert assembly in an opened position;
FIGS. 31-37 are perspective views of various components of an assembly that is a variation on the theme of the assembly of FIG. 30;
FIG. 38 is not an embodiment of the invention and shows a perspective view of a container and insert assembly in an opened position;
FIG. 39 is not an embodiment of the invention and shows a perspective view of a container and insert assembly;
FIG. 40 is not an embodiment of the invention and shows an exploded perspective view of a container and insert assembly;
FIG. 41 is not an embodiment of the invention and shows a perspective view of a container and insert assembly;
FIG. 42 is an exploded view of the assembly of FIG. 41;
FIG. 43 is not an embodiment of the invention and shows a perspective view of a container and insert assembly in an opened position;
FIG. 44 is an exploded view of the assembly of FIG. 43; and
FIG. 45 is not an embodiment of the invention and shows a perspective view of a container and insert assembly in an opened position.

### DETAILED DESCRIPTION OF THE PREFERRED EMBODIMENTS

In general, the present invention relates to containers and container inserts that store flat elongate articles, such as blood glucose test strips or other diagnostic strips, and facilitate easy access to such articles. As used herein, an "insert" may be a separate component that is inserted into a container, or alternatively, may be a structure that is integral with (e.g., molded as part of) the container. The preferred form of container according to the present invention is a vial, but other container types are contemplated. The term "article" as used herein refers to any flat elongate article, preferably a diagnostic test strip, more preferably a blood glucose test strip.

The various embodiments of the present invention as shown in the accompanying drawing figures each provide a solution to the problems presented by standard blood glucose test strip containers. Namely, the container assemblies described herein organize a plurality of strips in one or more arrays and position the strips such that they may be easily accessed and removed, e.g., one at a time, by a user.

Referring now in detail to the various figures of the drawings wherein like reference numerals refer to like parts, there are shown perspective views and sectional views of several container assemblies 10 according to the present invention. Each container assembly 10 includes a generally tubular container body 12 having a base 14, a sidewall 16 extending vertically from the base 14 and a dispensing opening 18, optionally opposite the base 14. The container body 12 includes an interior space 20 and optionally a desiccant material 22, e.g., a desiccant entrained polymer, communicating with the interior space 20. Each assembly 10 further includes a lid 24, which is vaulted so as to provide clearance for the ends of, e.g., stored diagnostic test strips when the lid 24 is closed on the container body 12. In other words, the ends of the stored articles protrude above the dispensing opening 18 when in storage position and into the vaulted space in the underside of the lid 24 when the lid 24 is closed. The lid 24 is optionally linked to the body 12 by an integral hinge 26. The hinge 26 can be configured to orient the lid 24 to seat on the body 12 when the lid 24 and body 12 are together. As illustrated in several figures, the integral hinge 26 can extend from the sidewall 16 of the body 12.

The lid 24 and body 12 include complementary sealing surfaces which, when the lid 24 is closed, provide a moisture tight seal. For example, sealing configurations that may be used according to the present invention include, but are not limited to: the lip-seal configuration disclosed in U.S. Pat. No. 7,537,137; or sealing configurations disclosed in U.S. Pat. Pub. No. 2011/0127269. Other moisture tight sealing configurations are contemplated as well.

As used herein, the terms "moisture tight" mean the moisture ingress of the container (after three days) was less than about 1500 micrograms of water, in another embodiment, about 500 micrograms of water, in a further embodiment, about 300 micrograms of water, in yet another embodiment, about 150 micrograms of water determined by the following test method: (a) place one gram plus or minus 0.25 grams of molecular sieve in the container and record the weight; (b) fully close the container; (c) place the closed container in an environmental chamber at conditions of 80% relative humidity and 22.2°C (72 F.); (c) after one day, weigh the container containing the molecular sieve; (d) after four days, weigh the container containing the molecular sieve; and (e) subtract the first day sample from the fourth day sample to calculate the moisture ingress of the container in units of micrograms of water.

The container assembly 10 is preferably resealable, i.e, the lid 24 can be opened/reopened and closed/reclosed numerous times (e.g. more than ten times) and the assembly 10 can still retain its moisture tight properties. Preferably, a moisture tight container assembly according to the present invention would admit less than 1,000 micrograms per day of water determined by a moisture ingress test method. The container optionally is sized as a pharmaceutical package (e.g., a vial) enclosing between 1 and 500 ml of interior volume, alternatively between 10 and 200 ml of interior volume, alternatively between 20 and 100 ml of interior volume.

The container assembly 10 further includes an insert 28 disposed within the interior space 20 of the container body 12. As described above, the insert 28 may be a separate component inserted within the interior space 20 or, alternatively, may be an integral part of (e.g., molded into) the container body 12. The insert 28 in any given embodiment retains a plurality of flat elongate articles 30 in a storage position (and in some instances, a separate retrieval position) that enables easy access to and removal of the articles, e.g., one at a time, by a user with debilitated fingers. The present invention includes optionally empty container assemblies as described herein, or optionally container assemblies as described herein containing a plurality of flat elongate articles (e.g., blood glucose test strips or other diagnostic strips).

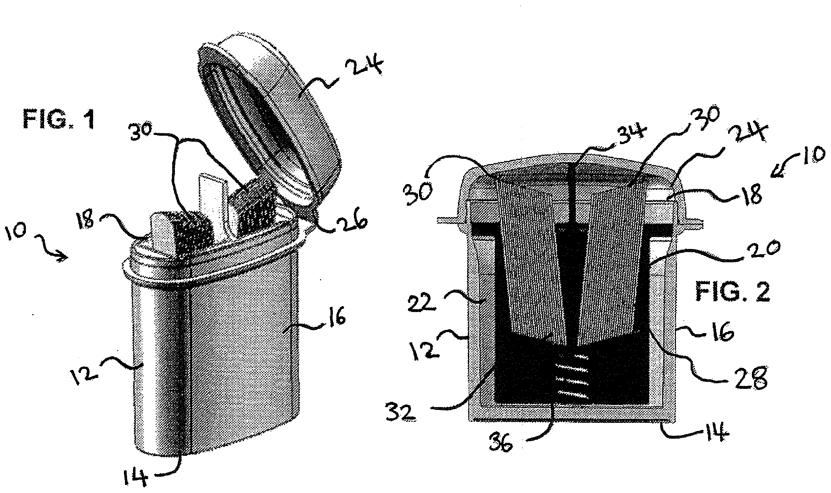

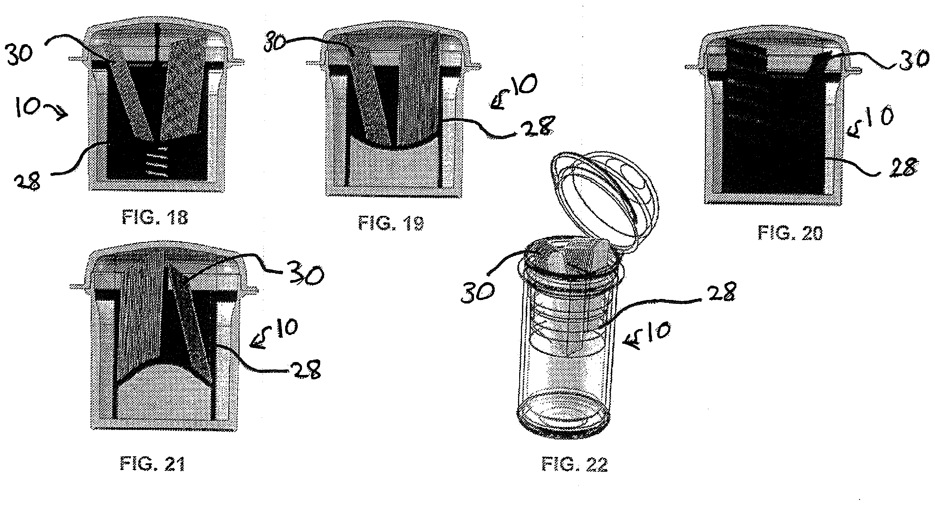

Referring to Figs. 1 and 2, the assembly 10 has an insert 28 which includes a v-shaped platform 32 for supporting the plurality of flat elongate articles 30 (e.g., diagnostic strips) in a generally vertically and slightly slanted storage position within the container body 12. Protruding upwards from the platform 32 is a divider 34 which compartmentalizes the plurality of articles 30 into two separate arrays. The divider 34 protrudes above the dispensing opening 18 and contacts the underside of the lid 24 when the lid 24 is closed. The articles 30 protrude above the dispensing opening 18 but the vaulted configuration of the lid 24 provides clearance so that the lid 24 does not contact or otherwise interfere with the articles 30. The assembly 10 further comprises a compression spring 36 disposed in the interior space 20 of the container body 12 between the base 14 and the underside of the platform 32. When the lid 24 on the assembly 10 is closed, the underside of the lid 24 presses down onto the top of the divider 34, causing the spring 36 to compress. When the lid is opened, the compression spring 36 is released, thus raising the platform 32 upwards. The raised platform provides improved accessibility to the articles 30 for a user. For example, each outer-most article 30 is slightly raised above the adjacent article, thus providing improved accessibility, especially for a person with debilitated fingers, e.g., a diabetic. Fig. 18 is a cross sectional view of the assembly 10 of Figs. 1 and 2 wherein some articles 30 in the array to the left of the divider 34 have been removed.

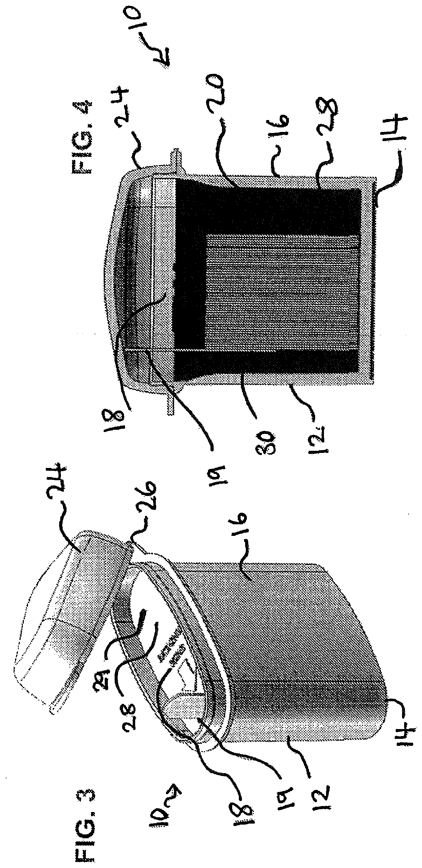

Referring to Figs. 3 and 4 there is shown a container assembly 10 including an insert 28 retaining a plurality of flat elongate articles 30, which are held in a generally vertical storage position. The insert 28 includes a ceiling 29, which provides an upper surface that blocks direct access to the articles 30 and prevents the articles 30 from falling out inadvertently, e.g., if the opened assembly 10 is held upside-down or knocked over. There is a slot 19 at one end of the ceiling 29 which provides egress to preferably one article 30 at a time. For example, a user may slightly tilt and shake the assembly 10 so as to align a single article 30 with the slot 19 until the article is accessible to the user to be manually pulled out.

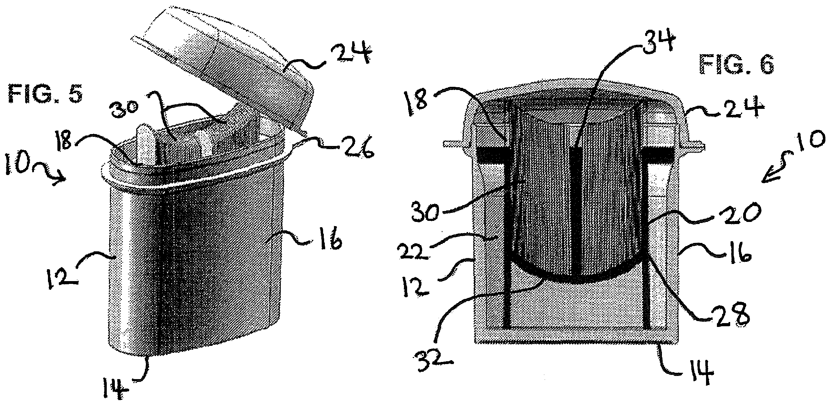

Referring to Figs. 5 and 6, there is shown a container assembly 10 having an insert 28 comprising a concave platform 32 and a fixed divider 34 compartmentalizing the plurality of flat elongate articles 30 into two separate arrays. The articles 30 are held in a generally vertical storage position. The patterns of the arrays render the outer-most article easily accessible to a user. Fig. 19 is a cross sectional view of the assembly 10 of Figs. 5 and 6 wherein some articles 30 in the array to the left of the divider 34 have been removed.

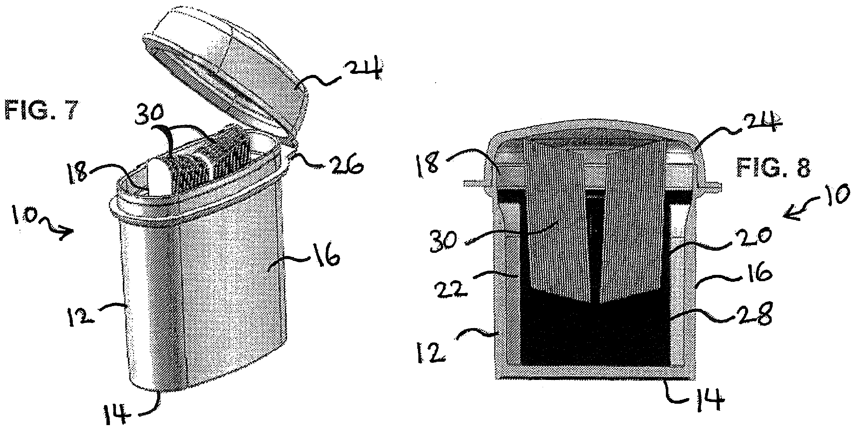

Referring to Figs. 7 and 8, there is shown a container assembly 10 having an insert 28 comprising a v-shaped platform 32 and a fixed divider 34 compartmentalizing the plurality of flat elongate articles 30 into two separate arrays. The articles 30 are held in a generally vertical and slightly slanted storage position. The patterns of the arrays render the outer-most article easily accessible to a user. Fig. 20 is a cross sectional view of the assembly 10 of Figs. 7 and 8 wherein some artices in the array to the right of the divider 34 have been removed.

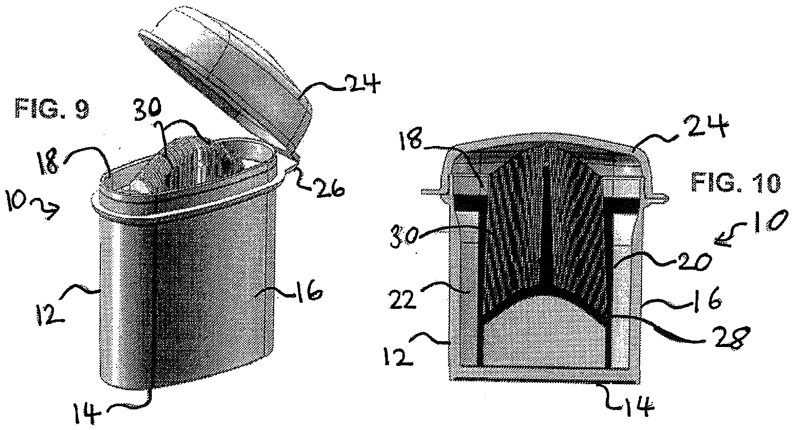

Referring to Figs. 9 and 10, there is shown a container assembly 10 having an insert 28 comprising a convex platform 32 and a divider 34 compartmentalizing the plurality of flat elongate articles 30 into two separate arrays. The articles 30 are held in a generally vertical and slightly slanted storage position. The patterns of the arrays render the outer-most article easily accessible to a user. Fig. 21 is a cross sectional view of the assembly 10 of Figs. 9 and 10 wherein some articles in the array to the right of the divider 34 have been removed.

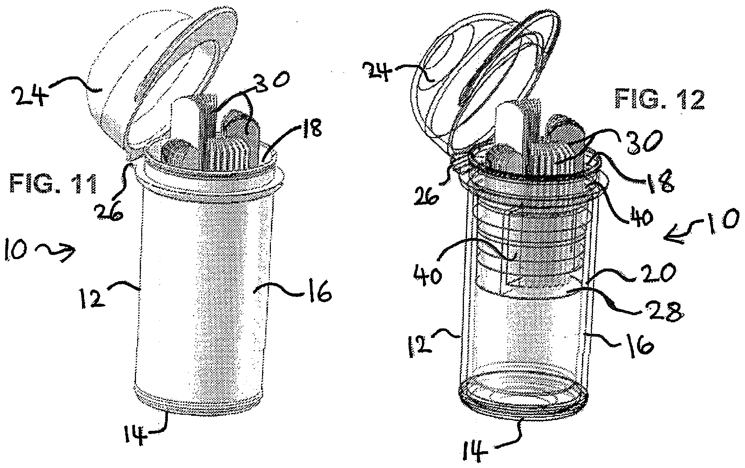

The containers disclosed herein are primarily either oblong (e.g., Figs. 1-10) or round (e.g., Figs. 11-15), however the present invention may also include containers having other geometries.

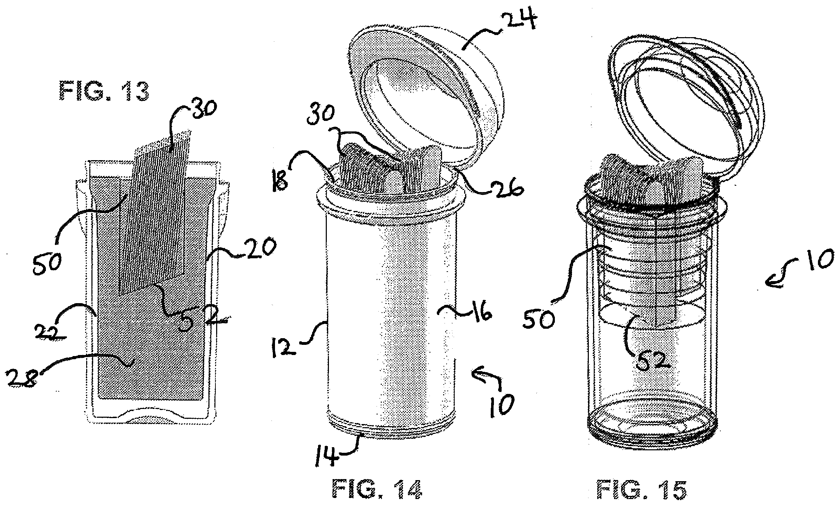

Referring to Figs. 11 and 12, there is shown a round container assembly 10 having an insert 28 comprising a plurality of compartments 40 of staggered depths, each compartment 40 containing, for example, an array of ten to twelve flat elongate articles 30. This configuratoin permits the generally vertically positioned arrays of articles 30 to rest at staggered heights to make them easier for a user to access and extract. In the embodiment shown, there are four compartments 40, however, more or fewer compartments 40 may be used according to the present invention, depending on design requirements. For example, Figs 13-15 show a round container assembly 10 having an insert comprising two compartments 50 of approximately equal depths. Each compartment includes a slightly slanted platform 52, which holds arrays of articles 30 in a generally vertical and slightly slanted storage position. Preferably, each platform 52 is slanted in a direction opposite the platform 52 of the adjacent compartment 50. In this way, one array of articles 30 is slanted in a direction opposite the other array of articles 30 to further ease a user's accessability to a single article 30 from an array. Fig. 22 is a cross sectional view of the assembly 10 of Figs. 13-15 wherein some articles 30 in the array in one of the compartments 50 have been removed.

Figs. 18-22, described above, illustrate how the inserts 28 may be designed to prevent the articles 30 (e.g., strips) from falling into a horizontal position, which would render removal thereof by a user very difficult.

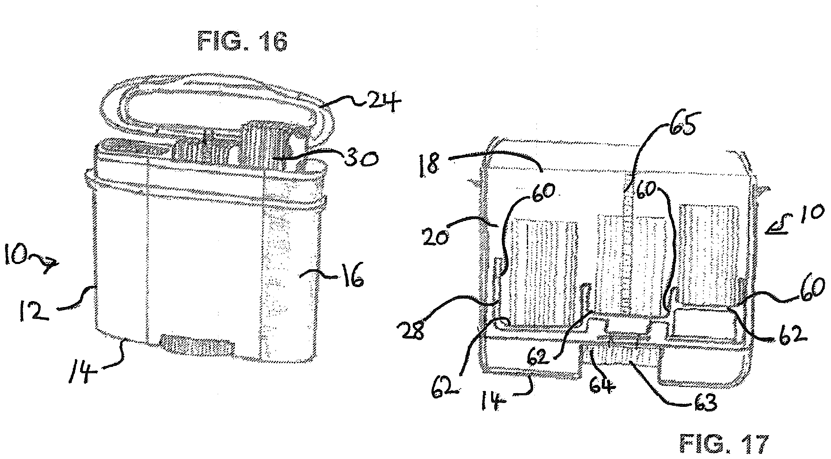

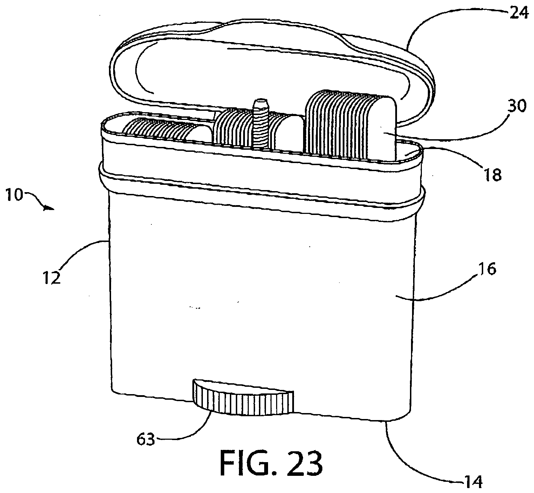

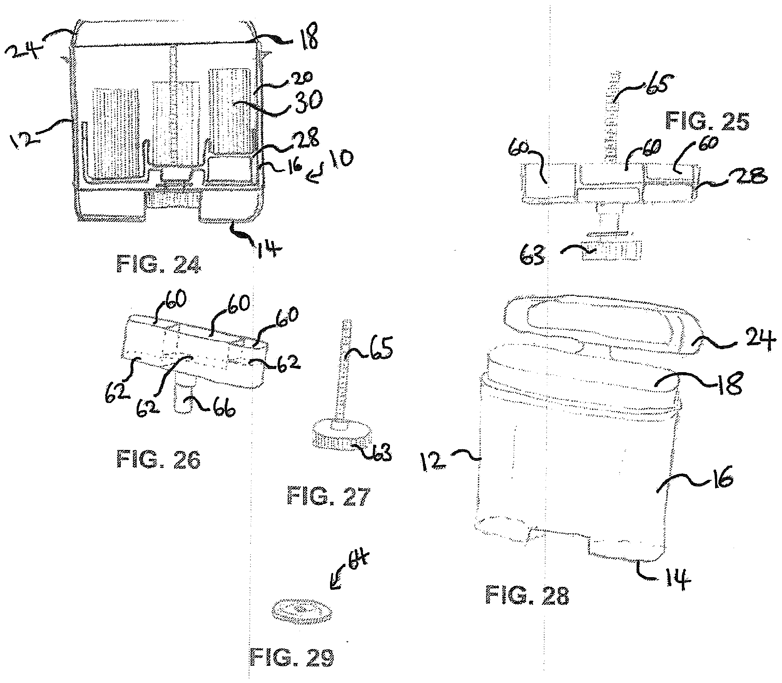

Figures 16, 17 and 23-29 show a multiple-component container assembly 10 for storing arrays of flat elongate articles. The container body 12 and lid 24 in the illustrated embodiment are generally oblong in shape. The insert 28 disposed within the container body 12 includes a plurality of compartments 60 - three compartments as shown in the illustrated embodiment, although more or fewer may be used depending on design requirements. Each compartment 60 includes a platform 62 at a height that differs from that of the platform 62 in the adjacent compartment 60. Preferably, the platform heights ascend in a stepped configuration from left to right or right to left.

The assembly 10 further includes a knob 63 disposed within a recess in the base 14 of the container body 12. The knob 63 is rigidly secured to a threaded shaft 65 which protrudes through a small opening optionally in the center of the base 14 and into the interior space 20 of the container body 12. Manual rotation of the knob 63 rotationally drives the shaft 65. The insert 28 includes a threaded tube 66. In assembled form, the shaft 65 threadedly engages the tube 66 of the insert 28. In use, rotation of the knob 63 drives the insert vertically, either upward or downward, depending on the direction of rotation. Preferably, a round seal 64, made e.g., from a thermo elastic polymer, is seated around the tube 66 above the base 14. The seal 64 protects the seal integrity of the container at the point where the shaft 65 enters the inner space 20 of the container body 12 through the base 14.

To use the assembly 10 of Figs. 16, 17 and 23-29, a user would open the lid 24 and rotate the knob 63 until the highest array of articles 30 is accessible for extraction. Each array is generally held in a vertical storage position and may contain, e.g., ten to twelve articles 30. The separate compartments 60 at different heights help to facilitate accessibility of the articles 30 and make it easier for a user to extract one article 30 at a time. When the articles 30 in the uppermost compartment 60 have been exhausted, the user may rotate the knob 63 until the array of articles 30 in the next highest compartment 60 are accessible.

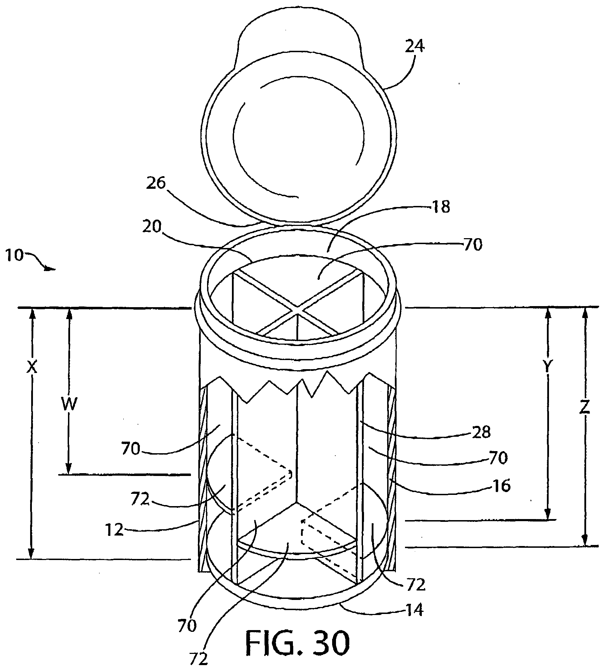

Referring to Fig. 30, there is shown a container assembly 10 having an insert 28 comprising four compartments 70. In the embodiment shown, the compartments 70 are of approximately equal dimensions in the transverse plane. Each compartment includes a platform 72 for supporting articles in a generally vertical storage position. Each platform 72 is at a different height (represented by dimensions w, x, y and z) from that of the platform 72 in the adjacent compartment 70. These platforms 72 of different hights provide staggered heights of arrays of articles (one array per compartment 70) to facilitate easy accessibility and extraction of the articles, e.g., one at a time. Each array may contain, for example, twelve or thirteen articles (e.g., diagnostic strips).

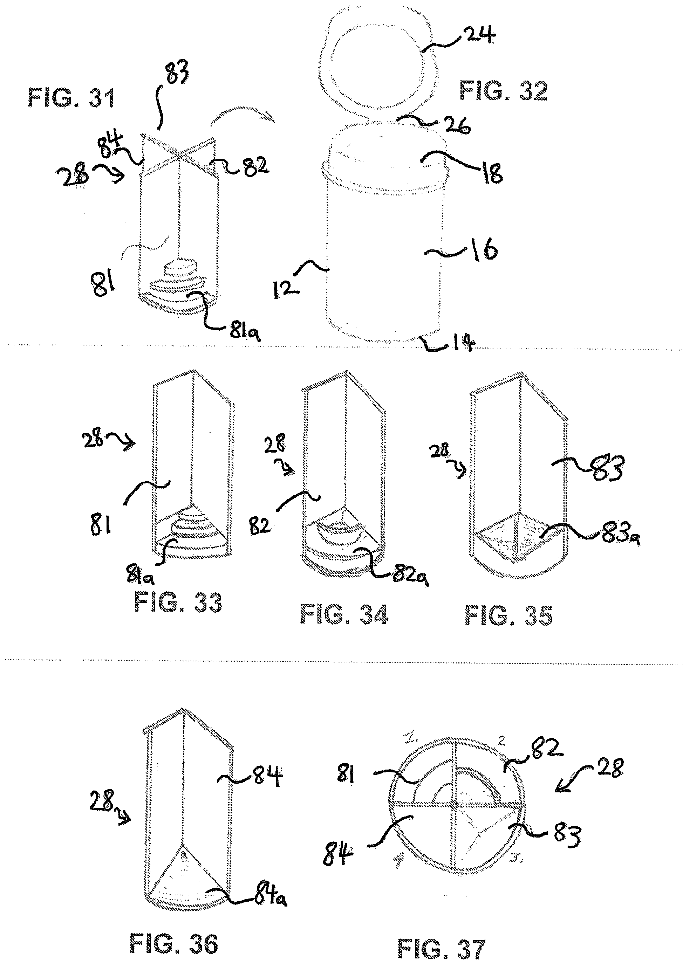

Figures 31-37 show alternative configurations of compartments (as shown, quadrants, however more or fewer than four compartments are contemplated) for an insert 28 similar to the insert 28 of Fig. 30. Each compartment or quadrant has a unique platform configuration. For example, compartment 81 has a step-like platform 81a, adapted to support an array of articles in a generally vertical storage position in ascending heights. Compartment 82 has a platform 82a with an arcuate divider. The platform 82a is adapted to support a split array of articles in a generally vertical storage position. Compartment 83 has a v-shaped platform 83a adapted to support an array of articles in a generally vertically and slightly slanted storage position. Compartment 84 has a horizontal, or alternatively inclined platform 84a adapted to support an array of articles in a generally vertically (and in the case of an inclined platform, slightly slanted) storage position. An insert 28 having compartments with varying platform configurations such as those shown in Figs. 33-37, improves accessibility of articles stored therein for extraction, e.g. one at a time, by a user.

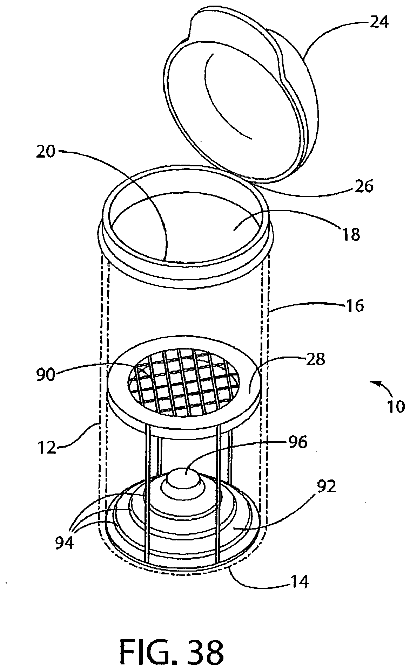

Referring to Fig. 38, there is shown a container assembly 10 having an insert 28 comprising a generally horizontal grid 90 disposed beneath the dispensing opening 18. The insert 28 futher includes a round platform 92 having ascending steps 94 which end at an upper, generally horizontal plane 96. The grid 90 is adapted to subdivide a plurality of articles into several different arrays. The stepped configuration of the platform 92 is adapted to stagger the heights of the articles and holds the articles in a generally vertical, and optionally slightly slanted storage position. The combination of the grid 90 and steps 94 on the platform 92 provides a storage position for articles stored in the container body 12 that facilitates easy access to the articles.

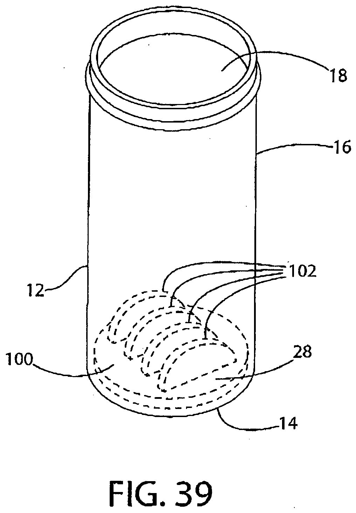

Referring to Fig. 39, there is shown a container body 12 having disposed therein an insert 28 comprising a platform 100 with a plurality of dividers 102. The dividers 102 are adapted to separate arrays of articles in a generally vertical and optionally slightly slanted storage position, to facilitate easy access to the articles for extraction, e.g., one at a time.

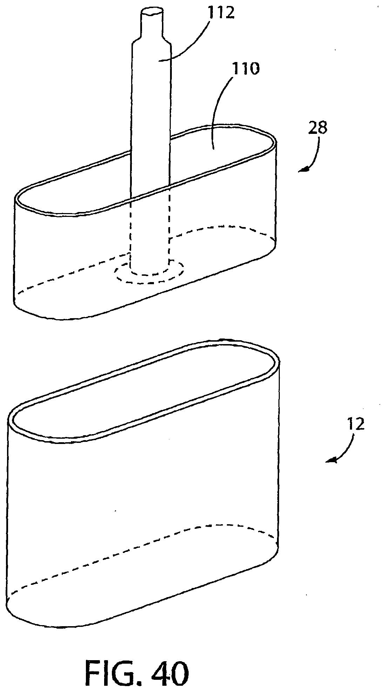

Referring to Fig. 40, there is shown a container body 12 and an insert 28 that is removable and replaceable into/out of the body 12 (multiple times) by a user. The insert 28 comprises a basket 110 adapted to hold a plurality of articles in a generally vertical and optionally slightly slanted storage position. The insert 28 further comprises a gripping post 112 secured thereto. The gripping post 112 enables a user to manually pull the insert 28 out of the container body 28 to access and extract an article and then place the insert 28 back into the container body 28 for storage. This configuration is especially helpful to users with seriously debilitated fingers. Furthermore, this configuration may be implemented in combination with any of the other insert configurations disclosed herein, such as inserts having dividers and stepped, slanted or hyperbolic platforms, etc.

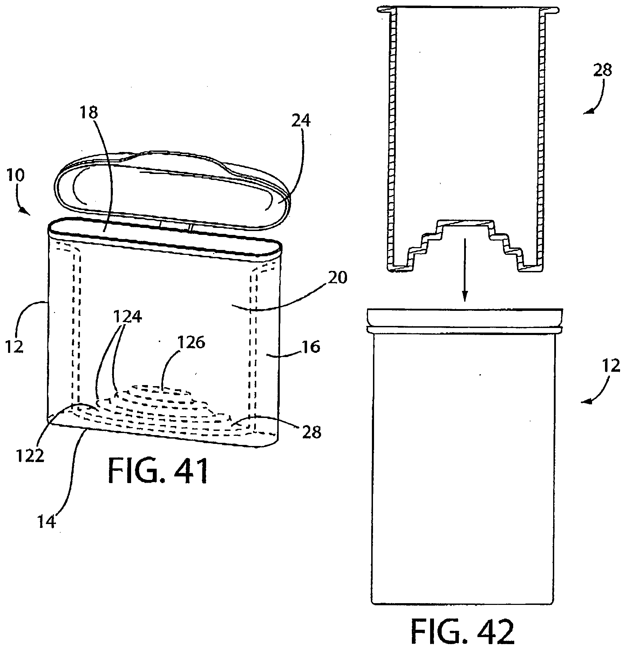

Referring to Figs. 41 and 42, there is shown a container assembly 10 comprising an insert 28 having a generally oblong platform 122 including ascending steps 124 which end at an upper, generally horizontal plane 126. This configuration is adapted to stagger articles in a generally vertical and optionally slightly slanted storage position, to facilitate easy access to the articles.

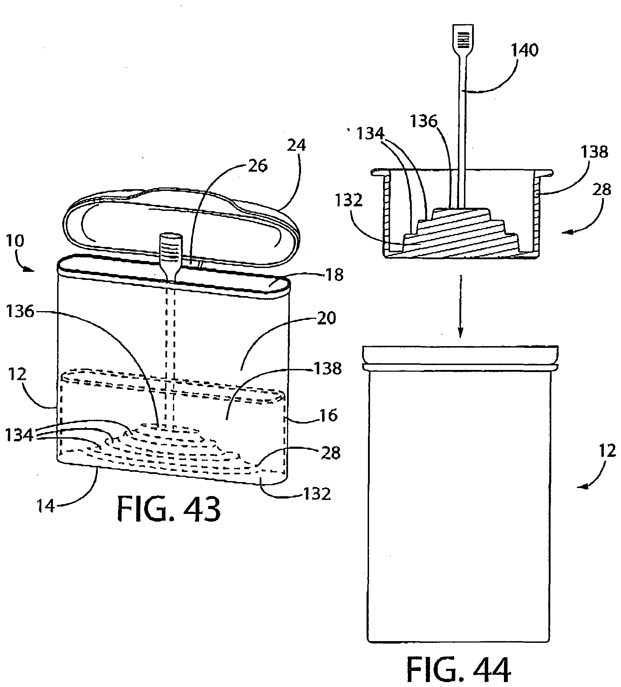

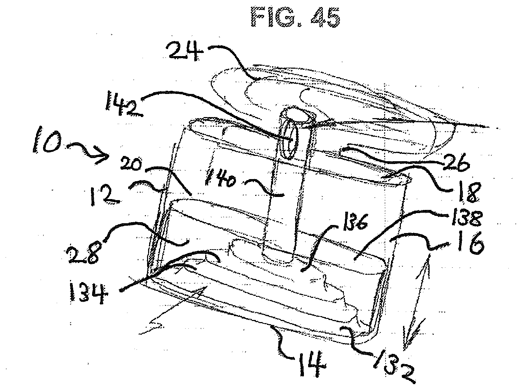

Referring to Figs. 43-45, there is shown a container assembly 10 comprising an insert 28 having a generally oblong platform 132 including ascending steps 134 which end at an upper, generally horizontal plane 136. The insert 28 comprises a basket 138 adapted to hold a plurality of articles in a generally vertical and optionally slightly slanted storage position. The insert 28 further comprises a gripping post 140 secured thereto. The gripping post 140 enables a user to manually pull the insert 28 out of the container body 12 to access and extract an article and then place the insert 28 back into the container body 12 for storage. Alternatively, the insert 28 can be elevated but is only partially removable from the container body 28. For example, a rim or other blocking structure may retain the insert 28 within the container body 12 to prevent the insert 28 from being removed entirely. Optionally, the platform 132 is made from rubber or another pliable and resilient material. With such a feature, the lid 24 when closed presses down on the gripping post 140 and substantially flattens the pliable platform 132, thereby lowering the heights of articles within the container body 12 to a storage position. When the lid is opened, the pliable and resilient platform 132 springs upward and raises articles to a heightened retrieval position, thereby rendering such articles easily accessible for extraction, e.g., one at a time. Optionally, as shown in Fig. 45, the gripping post 140 includes a hole or recess 142 adapted to receive one or more digits of a user. This feature may enable a user to achieve a stronger grip on the gripping post 140 to remove (in whole or in part) the insert 28.

The unifying theme of the various embodiments of the invention as described herein is the use of container inserts to facilitate the organization and configuration of arrays of flat elongate articles, e.g., diagnostic strips. Inserts according to the present invention hold such articles in a generally vertical (optionally, slightly slanted) storage position and facilitate accessability of the articles for extraction, e.g., one at a time, especially for persons with debilitated fingers, e.g., diabetics.

While the invention has been described in detail and with reference to specific examples thereof, it will be apparent to one skilled in the art that various changes and modifications can be made therein without departing from the scope of the appended claims.

## Claims

1. A container assembly (10) for storing one or more arrays of flat elongate articles (30), the assembly comprising:
• a container body (12) having a base (14), a sidewall (16) extending vertically from the base, a dispensing opening (18) and an interior space (20);
• a lid (24) having a vaulted underside optionally linked to the container body by a hinge (26), the lid configured to seat on the container body and cover the dispensing opening when the lid is closed, wherein the lid and container body comprise complementary sealing surfaces which, when the lid is closed, provide a moisture tight seal; and
• an insert (28) disposed within the interior space of the container body, the insert being adapted to hold at least one array of flat elongate articles (30) in a generally vertical and optionally slightly slanted storage position, the insert comprising a platform (32) adapted to hold a first flat elongate article in an array higher than an adjacent second flat elongate article in the same array, the platform comprising a surface geometry that is concave,
wherein the ends of the array of articles protrude above the dispensing opening when in the storage position and into the vaulted space in the underside of the lid when the lid is closed and wherein the platform is in a first position to hold at least one array of articles in a storage position when the lid is closed, the platform being vertically moveable to a higher second position when the lid is opened to hold the at least one array of articles in a retrieval position.

2. The container assembly (10) of claim 1, the insert (28) comprising at least one array of flat elongate articles (30) held in a storage position.

3. The container assembly (10) of claims 1 or 2, wherein the flat elongate articles (30) are blood glucose test strips.

4. The container assembly (10) of any preceding claim, wherein the insert (28) is at least partially removable to facilitate access to at least one array of flat elongate articles (30) held in the insert.

5. The container assembly (10) of any preceding claim, further comprising a desiccant material (22) communicating with the interior space (20).

## Patentansprüche

1. Behälteranordnung (10) zum Lagern von einer oder von mehreren Anordnungen von flachen länglichen Erzeugnissen (30), wobei die Anordnung Folgendes umfasst:
• einen Behälterkörper (12) mit einem Boden (14), einer Seitenwand (16), die sich senkrecht von dem Boden erstreckt, einer Ausgabeöffnung (18) und einem Innenraum (20);
• einen Deckel (24) mit einer gewölbten Unterseite, die optional mit dem Behälterkörper durch ein Scharnier (26) verbunden ist, wobei der Deckel konfiguriert ist, auf dem Behälterkörper zu sitzen und die Ausgabeöffnung zu bedecken, wenn der Deckel geschlossen ist, wobei der Deckel und der Behälterkörper komplementäre Abdichtungsoberflächen umfassen, die, wenn der Deckel geschlossen ist, eine feuchtigkeitsdichte Abdichtung bereitstellen; und
• einen Einsatz (28), der innerhalb des Innenraums des Behälterkörpers angeordnet ist, wobei der Einsatz angepasst ist, wenigstens eine Anordnung von flachen länglichen Erzeugnissen (30) in einer im Allgemeinen senkrechten und optional leicht schrägen Lagerungsposition zu halten, wobei der Einsatz eine Plattform (32) umfasst, die angepasst ist, ein erstes flaches längliches Erzeugnis in einer Anordnung höher als ein benachbartes zweites flaches längliches Erzeugnis in derselben Anordnung zu halten, wobei die Plattform eine Oberflächengeometrie umfasst, die schalenförmig ist,
wobei die Enden der Anordnung von Erzeugnissen über die Ausgabeöffnung herausragen, wenn sie in der Lagerungsposition sind, und in den gewölbten Raum in der Unterseite des Deckels hineinragen, wenn der Deckel geschlossen ist, und wobei die Plattform in einer ersten Position ist, um wenigstens eine Anordnung von Erzeugnissen in einer Lagerungsposition zu halten, wenn der Deckel geschlossen ist, wobei die Plattform senkrecht auf eine höhere zweite Position bewegt werden kann, wenn der Deckel geöffnet ist, um die wenigstens eine Anordnung von Erzeugnissen in einer Entnahmeposition zu halten.

2. Behälteranordnung (10) nach Anspruch 1, wobei der Einsatz (28) wenigstens eine Anordnung von flachen länglichen Erzeugnissen (30), die in einer Lagerungsposition gehalten werden, umfasst.

3. Behälteranordnung (10) nach Anspruch 1 oder 2, wobei die flachen länglichen Erzeugnisse (30) Blutglucoseteststreifen sind.

4. Behälteranordnung (10) nach einem der vorhergehenden Ansprüche, wobei der Einsatz (28) wenigstens teilweise entfernt werden kann, um einen Zugang zu wenigstens einer Anordnung von flachen länglichen Erzeugnissen (30), die in dem Einsatz gehalten werden, zu erleichtern.

5. Behälteranordnung (10) nach einem der vorhergehenden Ansprüche, ferner umfassend ein Trockenmittelmaterial (22), das mit dem Innenraum (20) in Berührung steht.

## Revendications

1. Ensemble contenant (10) pour stocker un ou plusieurs groupements d'articles allongés plats (30), l'ensemble comprenant :
• un corps de contenant (12) ayant une base (14), une paroi latérale (16) s'étendant à la verticale depuis la base, une ouverture de distribution (18) et un espace intérieur (20) ;
• un couvercle (24) ayant une face inférieure voûtée facultativement liée au corps de contenant par une charnière (26), le couvercle étant configuré pour s'appuyer sur le corps de contenant et couvrir l'ouverture de distribution lorsque le couvercle est fermé, dans lequel le couvercle et le corps de contenant comprennent des surfaces d'étanchéité complémentaires qui, lorsque le couvercle est fermé, fournissent un joint d'étanchéité étanche à l'humidité ; et
• une pièce rapportée (28) disposée au sein de l'espace intérieur du corps de contenant, la pièce rapportée étant adaptée pour maintenir au moins un groupement d'articles allongés plats (30) dans une position de stockage généralement verticale et facultativement légèrement inclinée, la pièce rapportée comprenant une plateforme (32) adaptée pour maintenir un premier article allongé plat dans un groupement plus haut qu'un second article allongé plat adjacent dans le même groupement, la plateforme comprenant une géométrie de surface qui est concave,
dans lequel les extrémités du groupement d'articles dépassent au-dessus de l'ouverture de distribution lorsqu'elles sont dans la position de stockage et dans l'espace voûté dans la face inférieure du couvercle lorsque le couvercle est fermé, et dans lequel la plateforme est dans une première position pour maintenir au moins un groupement d'articles dans une position de stockage lorsque le couvercle est fermé, la plateforme étant mobile à la verticale vers une seconde position plus haute lorsque le couvercle est ouvert pour maintenir l'au moins un groupement d'articles dans une position de récupération.

2. Ensemble contenant (10) selon la revendication 1, la pièce rapportée (28) comprenant au moins un groupement d'articles allongés plats (30) maintenus dans une position de stockage.

3. Ensemble contenant (10) selon les revendications 1 ou 2, dans lequel les articles allongés plats (30) sont des bandes réactives pour glycémie.

4. Ensemble contenant (10) selon l'une quelconque des revendications précédentes, dans lequel la pièce rapportée (28) est au moins partiellement amovible pour faciliter l'accès à au moins un groupement d'articles allongés plats (30) maintenus dans la pièce rapportée.

5. Ensemble contenant (10) selon l'une quelconque des revendications précédentes, comprenant en outre un matériau absorbeur d'humidité (22) communiquant avec l'espace intérieur (20).
